# EUROPÄISCHE PATENTSCHRIFT

(11) **EP 3 331 448 B1**
(45) Veröffentlichungstag und Bekanntmachung des Hinweises auf die Patenterteilung: **02.10.2019**
(21) Anmeldenummer: 16759986.9
(22) Anmeldetag: 05.08.2016
(51) Int. Cl.: A61B 6/03, A61B 6/04, A61B 6/08, A61B 6/14

(54) **VERFAHREN ZUR ERSTELLUNG EINER DENTALEN 3D-RÖNTGENAUFNAHME**
METHOD FOR CREATING A 3D DENTAL X-RAY IMAGE
PROCÉDÉ DE RÉALISATION D'UNE RADIOGRAPHIE DENTAIRE 3D

(30) Priorität: 06.08.2015 DE 102015215048
(43) Veröffentlichungstag der Anmeldung: 13.06.2018
(73) Patentinhaber: Sirona Dental Systems GmbH, 64625 Bensheim (DE)
(72) Erfinder: BECKHAUS, Christian, 64297 Darmstadt (DE)
(74) Vertreter: Özer, Alpdeniz
(86) Internationale Anmeldenummer: PCT/EP2016/068718
(87) Internationale Veröffentlichungsnummer: WO 2017/021520

(56) Entgegenhaltungen:
- DE-A1- 19 962 205
- US-A1- 2003 058 988

## Beschreibung

### Technisches Gebiet

Die Erfindung betrifft ein Verfahren zur Erstellung einer dentalen 3D-Röntgenaufnahme eines Kieferbereichs eines Patienten aus mehreren während eines Umlaufs eines Röntgenstrahlers und eines Röntgendetektors um einen Kopf des Patienten erzeugten Projektionsaufnahmen eines Aufnahmevolumens mit einem zylinderförmigen Kernvolumen mit einer Grundfläche, wobei der Patient so positioniert wird, dass sich der Kieferbereich innerhalb des Kernvolumens befindet.

### Stand der Technik

In bekannten dentalen Röntgengeräten zur Erstellung von Aufnahmen im Kiefer- oder Kopfbereich eines Patienten wird der Patient liegend, stehend oder sitzend positioniert. Zur Positionierung dient z.B. ein Aufbiss oder eine Stirnstütze.

Aus der DE 10 2008 035 412 A1 ist beispielsweise ein Verfahren und eine Vorrichtung zur Erzeugung einer 3D-Röntgenaufnahme bekannt, wobei ein Patient mittels eines Aufbisses zwischen einem Röntgenstrahler und einem Röntgendetektor positioniert wird und die Röntgenaufnahme durch einen Umlauf von Röntgenstrahler und Röntgendetektor um den positionierten Patienten erzeugt wird.

Aus der US 2003/0058988 A1 ist eine Positioniervorrichtung für das Vermessen eines Unterkiefers bekannt, wobei die Unterkante des Unterkiefers eines Patienten parallel zur Horizontalen ausgerichtet wird. Die Vorrichtung ermöglicht es, eine genau reproduzierbare Positionierung zu erreichen.

Aus der DE 199 62 205 A1 ist bekannt, bei der Bestimmung von Zahnachsenneigungen aus Röntgenaufnahmen eine Schieflage des Patientenkopfen relativ zu der Aufnahmevorrichtung zu berücksichtigen und zu korrigieren.

Das Aufnahmevolumen einer solchen Vorrichtung ist näherungsweise zylinderförmig mit horizontal verlaufender Grundfläche. Die Größe, insbesondere die Höhe, richtet sich im Wesentlichen nach der Größe bzw. Höhe des Detektors und des Röntgenstrahls und muss auf die Größe des Aufzunehmenden Objekts, also die Größe eines menschlichen Kiefers abgestimmt sein.

Die Aufgabe besteht nun darin, die aus dem Stand der Technik bekannten Verfahren weiterzubilden und insbesondere eine möglichst optimale Ausnutzung des Aufnahmevolumens bzw. eine Reduktion des notwendigen Aufnahmevolumens bei Darstellung des kompletten Kieferbereichs inklusive der Kiefergelenke zu ermöglichen.

### Darstellung der Erfindung

Ein Gegenstand der Erfindung ist ein Verfahren zur Erstellung einer dentalen 3D-Röntgenaufnahme eines Kieferbereichs eines Patienten aus mehreren während eines Umlaufs eines Röntgenstrahlers und eines Röntgendetektors um einen Kopf des Patienten erzeugten Projektionsaufnahmen eines Aufnahmevolumens mit einem zylinderförmigen Kernvolumen mit einer ebenen Grundfläche, wobei der Patient so positioniert wird, dass sich der Kieferbereich innerhalb des Kernvolumens befindet und eine Unterkante eines Unterkiefers des Patienten und die Grundfläche des Kernvolumens zumindest näherungsweise parallel zueinander ausgerichtet werden.

Ein typisches Röntgengerät zur Erzeugung einer dentalen 3D-Aufnahme weist ein durch den Umlauf von Röntgendetektor und Röntgenstrahler abgetastetes Aufnahmevolumen auf. Die Form des Aufnahmevolumens wird durch die Umlaufbahn von Detektor und Strahler sowie durch die beispielsweise trichterförmige Form und die Ausrichtung des Röntgenstrahls bedingt. Dies kann beispielsweise zu einem so genannten 100%-Aufnahmevolumen mit einer in Richtung eines Zentrums zunehmenden Höhe führen, z.B. einem zylinderförmigen Volumen mit einseitig oder beidseitig aufgesetzten Kegeln. Das Aufnahmevolumen kann in Abhängigkeit von dem Verwendeten Röntgengerät aber auch jede andere Form des Aufnahmevolumens aufweisen, z.B. kugelförmig, ellipsoid, kegelförmig etc.

Um den Kieferbereich eines Patienten zu vermessen, muss der Kieferbereich innerhalb des Aufnahmevolumens positioniert werden. Um die Strahlenbelastung möglichst gering zu halten, sollte hierbei die Größe des Aufnahmevolumens die Größe des Kieferbereichs nicht besonders übersteigen, auch wenn die Form des Aufnahmevolumens natürlich nicht unbedingt der Form des Volumens entspricht, welches der Kieferbereich einnimmt.

Mit Kernvolumen wird ein Teilvolumen dieses Aufnahmevolumens bezeichnet, welches einerseits noch vollständig innerhalb des Aufnahmevolumens liegt, also zur Vermessung verwendet werden kann und soll, und andererseits zylinderförmig ausgebildet ist, wobei die Grundfläche des Kernvolumens zumindest näherungsweise senkrecht zu der oder den Rotationsachsen des Röntgendetektors bzw. des Röntgenstrahlers ausgerichtet ist und als Referenz für die Ausrichtung des Patienten dient. Es sei angemerkt, dass das Kernvolumen die Form eines allgemeinen Zylinders hat, der eine ebene Grundfläche, eine der Grundfläche entsprechende, entlang einer Geraden relativ zur Grundfläche verschobene Deckfläche und eine diese beiden Flächen gerade verbindende Mantelfläche aufweist. Das Kernvolumen muss so groß sein, dass es den gesamten aufzunehmenden Bereich, also beispielsweise den gesamten Kieferbereich abdeckt. Es versteht sich, dass das Aufnahmevolumen idealerweise nicht viel größer als das Kernvolumen ist und das Kernvolumen im Idealfall zumindest theoretisch genau dem für die Vermessung zuverlässig verwendbaren Teil des Aufnahmevolumens des Röntgengeräts entspricht.

Röntgengeräte, deren Strahler und Detektor sich um eine vertikale Achse drehen, weisen ein Kernvolumen mit einer zumindest näherungsweise horizontal verlaufenden Grundfläche auf. Der Patient wird sitzend oder stehend zwischen Röntgenstrahler und Röntgendetektor positioniert.

Andere Röntgengeräte ermöglichen eine liegende Positionierung des Patienten, wobei Strahler und Detektor entsprechend um eine zumindest näherungsweise horizontale Achse kreisen und die Grundfläche das zylinderförmige Kernvolumen zumindest näherungsweise vertikal verläuft.

Bei einer normalen geraden Kopfhaltung eines stehenden oder sitzenden Patienten verläuft die Unterkante des Unterkiefers leicht schräg vom Kinn zum Hinterkopf hin ansteigend, während die Okklusionsebene im Wesentlichen horizontal verläuft. Wird der Kopf überstreckt, also nach hinten geneigt, so kann die Unterkante des Unterkiefers parallel zu einer horizontalen Ebene ausgerichtet werden, während die Okklusionsebene schräg verläuft.

Eine Ausrichtung der Unterkante des Unterkiefers parallel zur Grundfläche des Kernvolumens, die durch ein Überstrecken des Kopfes erreicht wird, ermöglicht eine optimale Ausnutzung des Kernvolumens, da der Kieferbereich in einer überstreckten Kopfhalten ein Volumen einnimmt, welches einem zylinderförmigen Volumen mit horizontaler Grundfläche besonders nahe kommt.

Ein Vorteil dieser Positionierung ist, dass die Höhe des Kernvolumens gering gehalten und trotzdem der gesamte Kieferbereich mit dem Kiefergelenk und gegebenenfalls auch der Kieferhöhle aufgenommen werden kann. Ferner ist der Kieferbereich durch eine solche Ausrichtung so im Kernvolumen positioniert, dass das Aufnehmen weiterer, eventuell störender Strukturen, wie der Halswirbelsäule weitgehend vermieden wird. Weiterhin wird durch diese Positionierung erreicht, dass die besonders strahlungsempfindlichen Organe Augen und Schilddrüse weitestgehend außerhalb des bestrahlten Volumens liegen, wodurch die Effektivdosis einer Aufnahme deutlich reduziert wird.

Erfindungsgemäß wird ein erster Winkel einer Okklusionsebene und/oder eines Kiefergelenks des positionierten Patienten relativ zu der Grundfläche oder einer Mantelfläche des Kernvolumens bestimmt und eine Lagekorrektur der 3D-Röntgenaufnahme um den ersten Winkel vorgenommen. Da die Okklusionsebene, also die Bissfläche bei normaler Kopfhaltung eines stehenden oder sitzenden Patienten im Wesentlichen horizontal verläuft, gibt der erste Winkel zwischen der Okklusionsebene eines positionierten Patienten zu einer horizontal verlaufenden Grundfläche eines für stehende oder sitzende Positionierung konzipierten Röntgengeräts die Abweichung der Position des Patienten von der normalen Kopfhaltung an. Entsprechendes gilt für eine liegende Positionierung und eine vertikal verlaufende Grundfläche des Kernvolumens. Durch das Vornehmen einer Lagekorrektur um den ersten Winkel kann die 3D-Röntgenaufnahme in einer normalen Kopfhaltung dargestellt werden. So kann trotz einer überstreckten Positionierung des Kopfes während der Aufnahme eine gewohnte Ansicht des Kiefers in der erhaltenen 3D-Röntgenaufnahme bereitgestellt werden. Diese Lagekorrektur kann direkt bei bzw. während der Rekonstruktion oder auch erst anschließend für die Darstellung der rekonstruierten Daten in einem Anzeigeprogramm vorgenommen werden. Vorteilhafterweise wird ein erster Winkel einer Okklusionsebene oder eines Kiefergelenk des positionierten Patienten relativ zu der Grundfläche oder einer Mantelfläche des Kernvolumens bestimmt und eine Lagekorrektur der 3D-Röntgenaufnahme um einen zweiten Winkel vorgenommen wird, der eine feste Differenz zu dem ersten Winkel aufweist. Besteht besonderes Interesse an einem Teilbereich des zu vermessenden Kieferbereichs, z.B. das Gaumendach oder der Sinusboden, so kann dies durch einen Offset bei der Lagekorrektur berücksichtigt werden, so dass die dargestellten Daten einer Aufnahme eines leicht nach vorn oder nach hinten geneigten Kopfes entsprechen.

Vorteilhafterweise wird zur Positionierung ein mechanisches Positioniermittel, insbesondere ein Aufbiss und/oder eine Stirnstütze und/oder eine Kinnauflage und/oder eine Auflage für die Nasenwurzel verwendet. Als Positioniermittel kann jedes bekannte Positioniermittel eingesetzt werden. Ein Aufbiss ist beispielsweise ein mechanisches Positioniermittel, welche einen von einem Patienten in den Mund zu nehmenden Bereich aufweist. Dies kann beispielsweise ein Okklusalaufbiss sein. Der Aufbiss ermöglicht sowohl ein einfaches Positionieren als auch ein einfaches Bestimmen des Winkels der Abweichung von einer normalen Kopfhaltung.

Vorteilhafterweise wird zur Positionierung eine Licht- oder Laserbereichsanzeige verwendet. Auch mittels einer Licht- oder Laserbereichsanzeige, also auf den Patientenkopf projizierten Laserlinien, kann das Positionieren des Patienten einfach und für den Patienten angenehm vorgenommen bzw. kontrolliert werden.

Die Bestimmung des Winkels der Abweichung von einer normalen Kopfhaltung kann z.B. über einen aus vielen Aufnahmedaten gewonnenen statistischen Mittelwert oder eine Bestimmung der Okklusalebene im rekonstruierten Datensatz mit Hilfe geeigneter Algorithmen, wie z.B. Segmentierung der Kauflächen, erfolgen.

Vorteilhafterweise ist die Licht- oder Laserbereichsanzeige ein Licht- oder Laservisier.

### Kurzbeschreibung der Zeichnungen

Ausführungsbeispiele der Erfindung sind in der Zeichnung dargestellt. Es zeigt die
- Fig. 1A,B: das Positionieren eines Patienten mittels eines Aufbisses und die
- Fig. 2A,B: das Positionieren eines Patienten mittels einer Laserbereichsanzeige.

### Ausführungsbeispiele

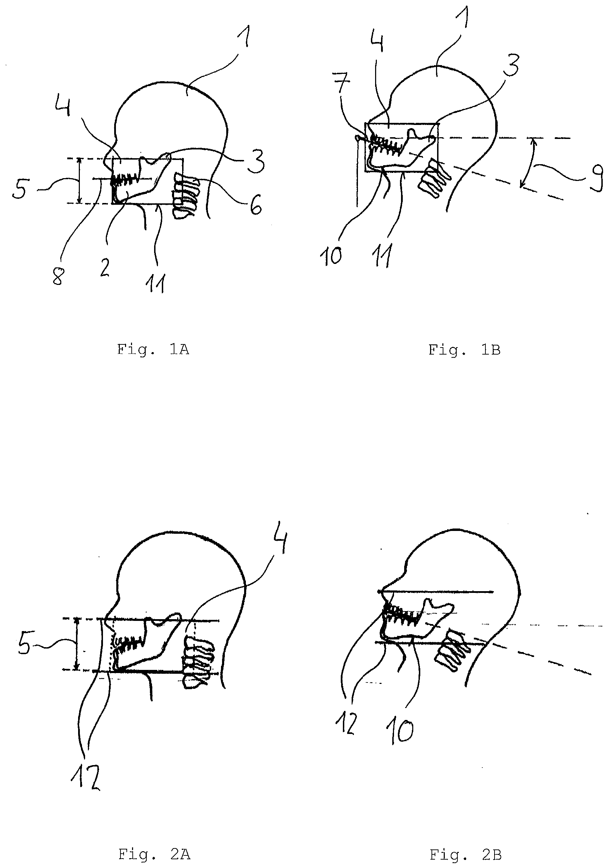

Die Fig. 1A zeigt schematisch einen Patientenkopf 1 in einer natürlichen Kopfhaltung, in dem ein Unterkiefer 2 und ein Kiefergelenk 3 skizziert sind. Ein zylinderförmiges Kernvolumen 4 eines Aufnahmevolumens (nicht dargestellt) mit einer Höhe 5 und einer Grundfläche 11 ist durch seitliche Begrenzungslinien dargestellt und deckt den Unterkiefer 2 zumindest teilweise ab. Eine Okklusionsebene 8 des Unterkiefers 2 verläuft näherungsweise parallel zur Grundfläche 11 des Kernvolumens 4.

Um den gesamten Unterkiefer 2, insbesondere auch das Kiefergelenk 3 und bevorzugt auch einen Oberkiefer (nicht dargestellt) aufzunehmen, müsste das Kernvolumen 4 bei dieser dargestellten Kopfhaltung bzw. Ausrichtung des Kopfes vergrößert werden. Je nach Größe bzw. Tiefe des Kernvolumens 4 werden allerdings auch andere Strukturen mit aufgenommen, welche die Aufnahmequalität der 3D-Röntgenaufnahme stören können oder besonders empfindlich gegenüber Röntgenstrahlung sind. Wie in Fig. 1A skizziert, wird bei einer natürlichen Kopfhaltung und einer zu großen Tiefe des Kernvolumens 4 ein Teil der Halswirbelsäule 6 mit aufgenommen.

In Fig. 1B ist der Kopf 1 des Patienten aus der natürlichen Kopfhaltung in eine überstreckte Haltung gebracht worden. Hierfür kann, wie in Fig. 1B dargestellt, ein Aufbiss 7 verwendet werden, der die Okklusionsebene 8 in einem ersten Winkel 9 zur Grundfläche 11 ausrichtet. Der erste Winkel 9 wird so eingestellt, dass eine Unterkante 10 des Unterkiefers 2 möglichst parallel zur Grundfläche 11 des zylinderförmigen Kernvolumens 4 ausgerichtet ist. Eine Höhe des Aufbisses 7 wird so eingestellt, dass die Unterkante 10 auf Höhe der Grundfläche 11 oder etwas darüber positioniert ist.

In dieser überstreckten Positionierung des Kopfes 1 reicht das Kernvolumen 4 aus, um den gesamten Kieferbereichs aus Oberkiefer (nicht dargestellt) Unterkiefer 2 und Kiefergelenk 3 zu erfassen. Eine Vergrößerung hinsichtlich der Höhe 5 ist nicht notwendig. Hierdurch kann die Strahlenbelastung für den Patienten gering gehalten werden. Ferner kann durch die erfindungsgemäße Positionierung das Aufnehmen störender Strukturen, wie z.B. der Halswirbelsäule vermieden werden. Geringere Anforderungen an die maximale Höhe 5 des Kernvolumens 4 und entsprechend des gesamten Aufnahmevolumens ermöglichen auch die Verwendung einer kostengünstigeren Aufnahmevorrichtung bzw. geringere Herstellungskosten von Röntgenstrahler und Detektor (nicht dargestellt).

In Fig. 2A und 2B ist das Positionieren des Patientenkopfes 1 mittels einer Laserbereichsanzeige 12 skizziert. Für die Positionierung werden mittels eines Lasers oder einer anderen Lichtquelle (nicht dargestellt), beispielsweise mittels eines Licht- oder Laservisiers, als Laserbereichsanzeige 12 zwei Linien auf den Patientenkopf 1 projiziert, welche die Höhe 5, den Verlauf der Grundfläche 11 und die räumliche Position des Kernvolumens 4 (durch gepunktete Linien skizziert) anzeigen. Der Patientenkopf 1 wird so ausgerichtet, dass die Unterkante 10 des Unterkiefers 2 näherungsweise parallel zur unteren Linie der Laserbereichsanzeige 12 verläuft.

Als weitere Hilfestellung für die Positionierung kann eine mittig zwischen den beiden Linien verlaufende weitere Linie projiziert werden, die bei einer optimalen Abstimmung von Position und Größe von aufzunehmendem Unterkiefer und Kernvolumen von einer Lippenspalte des Patienten zu einem Gehörgang des Patienten verlaufen sollte.

### Bezugszeichenliste

- 1: Patientenkopf
- 2: Unterkiefer
- 3: Kiefergelenk
- 4: Kernvolumen
- 5: Höhe des Kernvolumens
- 6: Halswirbelsäule
- 7: Aufbiss
- 8: Okklusionsebene
- 9: erster Winkel
- 10: Unterkante des Unterkiefers
- 11: Grundfläche des Kernvolumens
- 12: Laserbereichsanzeige

## Patentansprüche

1. Verfahren zur Erstellung einer dentalen 3D-Röntgenaufnahme eines Kieferbereichs eines Patienten aus mehreren während eines Umlaufs eines Röntgenstrahlers und eines Röntgendetektors um einen Kopf (1) des Patienten erzeugten Projektionsaufnahmen eines Aufnahmevolumens mit einem zylinderförmigen Kernvolumen (4) mit einer ebenen Grundfläche (11), wobei der Patient relativ zu dem Kernvolumen (4) so positioniert wird, dass sich der Kieferbereich innerhalb des Kernvolumens (4) befindet, und dass die Unterkante (10) eines Unterkiefers (2) des Patienten und die Grundfläche (11) des Kernvolumens (4) zumindest näherungsweise parallel ausgerichtet werden, **dadurch gekennzeichnet, dass** ein erster Winkel (9) einer Okklusionsebene (8) oder eines Kiefergelenks des positionierten Patienten relativ zu der Grundfläche oder einer Mantelfläche des Kernvolumens (4) bestimmt und eine Lagekorrektur der 3D-Röntgenaufnahme um den ersten Winkel (9) vorgenommen wird.

2. Verfahren nach Anspruch 1, **dadurch gekennzeichnet, dass** ein erster Winkel (9) einer Okklusionsebene (8) oder eines Kiefergelenk des positionierten Patienten relativ zu der Grundfläche oder einer Mantelfläche des Kernvolumens (4) bestimmt und eine Lagekorrektur der 3D-Röntgenaufnahme um einen zweiten Winkel vorgenommen wird, der eine feste Differenz zu dem ersten Winkel (9) aufweist.

3. Verfahren nach einem der Ansprüche 1 bis 2, **dadurch gekennzeichnet, dass** zur Positionierung ein mechanisches Positioniermittel verwendet wird.

4. Verfahren nach Anspruch 3, **dadurch gekennzeichnet, dass** das mechanische Positioniermittel ein Aufbiss (7) und/oder eine Stirnstütze und/oder eine Kinnauflage und/oder eine Auflage für die Nasenwurzel ist.

5. Verfahren nach einem der Ansprüche 1 bis 4, **dadurch gekennzeichnet, dass** zur Positionierung eine Licht- oder Laserbereichsanzeige (12) verwendet wird.

6. Verfahren nach Anspruch 5, **dadurch gekennzeichnet, dass** die Licht- oder Laserbereichsanzeige ein Licht- oder Laservisier umfasst.

## Claims

1. Method for creating a dental 3D X-ray image of a jaw region of a patient from a plurality of projection images of a recording volume having a cylindrical core volume (4) with a flat base (11) generated during a revolution of an X-ray emitter and an X-ray detector about the head (1) of a patient, wherein the patient is positioned relative to the core volume (4) such that the jaw region is located within the core volume (4) and such that the lower edge (10) of a lower jaw (2) of the patient and the base (11) of the core volume (4) are aligned at least approximately parallel, **characterized in that** a first angle (9) of an occlusal plane (8) or a temporomandibular joint of the positioned patient relative to the base or a lateral surface of the core volume (4) is determined and a positional correction of the 3D X-ray image by the first angle (9) is carried out.

2. Method according to Claim 1, **characterized in that** a first angle (9) of an occlusal plane (8) or a temporomandibular joint of the positioned patient relative to the base or a lateral surface of the core volume (4) is determined and a positional correction of the 3D X-ray image by a second angle, which displays a fixed difference to the first angle (9), is carried out.

3. Method according to any of Claims 1 through 2, **characterized in that** a mechanical positioning means is used for positioning.

4. Method according to Claim 3, **characterized in that** the mechanical positioning means is a bite block (7) and/or a forehead support and/or a chin support and/or a support for the nasal root.

5. Method according to any of Claims 1 through 4, **characterized in that** a light or laser range indicator (12) is used for the positioning.

6. Method according to Claim 5, **characterized in that** the light or laser range indicator comprises a light or laser sight.

## Revendications

1. Procédé pour la génération d'une radiographie 3D dentaire d'une zone de mâchoire d'un patient à partir de plusieurs d'enregistrements de projection d'un volume d'enregistrement comprenant un volume de base (4) cylindrique comprenant une surface de base plane (11) générés pendant une rotation d'un émetteur de rayons X et d'un détecteur de rayons X autour d'une tête (1) du patient, le patient étant positionné de telle façon par rapport au volume de base (4) que la zone de mâchoire se trouve à l'intérieur du volume de base (4), et que le bord inférieur (10) d'une mâchoire inférieure (2) du patient et la surface de base (11) du volume de base (4) sont orientés au moins approximativement parallèlement, **caractérisé en ce qu'**un premier angle (9) d'un plan d'occlusion (8) ou d'une articulation de mâchoire du patient positionné par rapport à la surface de base ou à une surface d'enveloppe du volume de base (4) est déterminé et qu'une correction de position de la radiographie 3D de la valeur du premier angle (9) est réalisée.

2. Procédé selon la revendication 1, **caractérisé en ce qu'**un premier angle (9) d'un plan d'occlusion (8) ou d'une articulation de mâchoire du patient positionné par rapport à la surface de base ou à une surface d'enveloppe du volume de base (4) est déterminé et une correction de position de la radiographie 3D de la valeur d'un deuxième angle, présentant une différence fixe par rapport au premier angle (9), est réalisée.

3. Procédé selon l'une quelconque des revendications 1 à 2, **caractérisé en ce qu'**un moyen de positionnement mécanique est utilisé pour le positionnement.

4. Procédé selon la revendication 3, **caractérisé en ce que** le moyen de positionnement mécanique est une pièce à mordre occlusale (7) et/ou un appui frontal et/ou un appui-menton et/ou un appui pour la racine du nez.

5. Procédé selon l'une quelconque des revendications 1 à 4, **caractérisé en ce qu'**un affichage de zone lumineux ou par laser (12) est utilisé pour le positionnement.

6. Procédé selon la revendication 5, **caractérisé en ce que** l'affichage de zone lumineux ou par laser comprend un viseur lumineux ou laser.
